# EUROPÄISCHE PATENTSCHRIFT

(11) **EP 0 458 949 B1**
(45) Veröffentlichungstag und Bekanntmachung des Hinweises auf die Patenterteilung: **03.05.1995**
(21) Anmeldenummer: 91901723.6
(22) Anmeldetag: 19.12.1990
(51) Int. Cl.: A61K 47/12, A61K 47/18, A61K 47/20, A61K 47/22, A61K 47/26, A61K 38/49

(54) **STABILISIERUNG VON K1K2P PRO**
STABILIZATION OF K1K2P PRO
STABILISATION DE K1K2P PRO

(30) Priorität: 20.12.1989 DE 3942144
(43) Veröffentlichungstag der Anmeldung: 04.12.1991
(73) Patentinhaber: BOEHRINGER MANNHEIM GMBH, 68298 Mannheim (DE)
(72) Erfinder: KOHNERT, Ulrich, D-8121 Habach (DE); RAINER, Rudolph, D-8120 Weilheim (DE)
(74) Vertreter: Huber, Bernhard, Dipl.-Chem.
(86) Internationale Anmeldenummer: EP9002248
(87) Internationale Veröffentlichungsnummer: WO9108763

(56) Entgegenhaltungen:
- EP-A- 0 211 592
- EP-A- 0 228 862
- EP-A- 0 297 294
- DE-A- 3 923 339
- Testvorschrift der Fa. Boehringer Mannheim Best. Nr. 1080954 ("t-PA Standard")
- BIOCHEMISTRY,VOL 28, AMERICAN CHEMICAL SOCIETY 1989,R.F. KELLY ET AL.

## Beschreibung

Der menschliche Gewebe-Plasminogen-Aktivator (t-PA) besitzt eine große therapeutische Bedeutung bei der Auflösung von Blutgerinnseln, z.B. bei Herzinfarkten. t-PA bewirkt die Auflösung von Blutgerinnseln durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, E, K1, K2 und P zusammengesetzt. Die Domäne P beinhaltet das proteolytisch aktive Zentrum, das die Spaltung von Plasminogen zu Plasmin bewirkt. Die gentechnologische Herstellung von t-PA oder verschiedenen t-PA-Muteinen, bei denen eine oder mehrere der Domänen F, E, K1 und K2 deletiert sind, in eukaryontischen und prokaryontischen Zellen ist bereits bekannt. Dabei werden t-PA-Derivate aus Prokaryonten im Gegensatz zu natürlichem t-PA in nicht glykosylierter Form synthetisiert.

Weiterhin ist bekannt, daß der Zuckeranteil einen erheblichen Einfluß auf die Löslichkeit und Aggregation von Proteinen hat (J. Biol. Chem. 263 (1988), 8832-8837). Es wurde nun gefunden, daß das nicht glykosylierte t-PA-Derivat K1K2P pro wesentlich schlechter löslich als glykosylierter t-PA ist.

K1K2P pro löst sich nicht in den üblicherweise zur Solubilisierung von Proteinen verwendeten Puffern, wie z.B. 50 mmol/l Na-Citrat, 50 mmol/l Phosphat oder physiologische NaCl-Lösung. Für den Einsatz als therapeutischer Wirkstoff sollte jedoch K1K2P pro mit einer höheren enzymatischen Aktivität von mindestens 0,4 MU/ml, vorzugsweise 0,4 MU/ml bis 10 MU/ml vorliegen. Die Einheit U ist dabei nach WHO, National Institute for Biological Standards and Control (vgl. H. Lill, ZGIMAL 42 (1987), 478-486) definiert.

Aus der EP-A 0 217 379 ist bekannt, die Löslichkeit von t-PA aus prokaryontischen Organismen (tPA pro) durch neutrale oder leicht alkalische Arginin-Formulierungen zu erhöhen. Ein Nachteil dieses Verfahrens ist jedoch, daß gute Löslichkeiten von t-PA pro nur mit sehr hohen Arginin-Konzentrationen erreicht werden können.

EP-A-0 228 862 offenbart eine pharmazeutische Zusammensetzung, die eine pharmazeutisch wirksame Menge von humanem Gewebsplasminogenaktivator und einen pharmazeutisch akzeptablen, Argininionen enthaltenden Puffer enthält, wobei die Zusammensetzung eine Chloridionen-Konzentration von weniger als etwa 0,3 M enthält.

EP-A-0 211 592 offenbart eine sterile pharmazeutische Dosierungseinheit von t-PA zur parenteralen Verabreichung, die 0,1 - 15 mg/ml t-PA in einer pharmazeutisch akzeptablen gepufferten wäßrigen Lösung bei pH 3,5 - 5,5 enthält. Nicht-glykosylierte t-PA-Muteine sind in diesem Dokument nicht beschrieben.

EP-A-0 361 475 beschreibt ein Präparat von rekombinantem, in Prokaryonten exprimiertem p-t-PA, und bestimmt unter anderem dessen löslichkeit in Abhängigkeit vom pH-Wert in beginwart von 0,3 mol/l Arginin-HCl plus 20 mmol/l zitronensäure.

WO-A-91/08764 mit einer Priorität vom 20. Dezember 1989 offenbart ein pharmazeutisches Präparat eines Proteins mit t-PA-Aktivität mit einer enzymatischen Aktivität von mindestens 0,25 MU/ml und einem pH-Wert von 4,5 - 9, das eine Substanz aus der Gruppe Zitronensäure, Ascorbinsäure, 2-Oxoglutarsäure, Fumarsäure, Tris und EDTA in einer Konzentration von mindestens 0,2 mol/l enthält, sowie ein Arzneimittel auf Basis eines Proteins mit t-PA-Aktivität und Verfahren zu seiner Herstellung.

Aufgabe der Erfindung war es demnach, pharmazeutische Präparate zu entwickeln, die K1K2P pro mit einer Aktivität von mindestens 0,4 MU/ml enthalten, wobei das t-PA-Derivat über einen längeren Zeitraum hinweg stabil sein soll.

Die erfindungsgemäße Aufgabe wird gelöst durch ein pharmazeutisches Präparat eines nicht glykosylierten t-PA-Derivats K1K2P pro mit einer enzymatischen Aktivität von mindestens 0,4 MU/ml mit einem pH-Wert von 4,5 bis 6,5, wobei diese Zusammensetzung Citrat und mindestens eine Verbindung aus der aus
a) Ascorbinsäure,
b) EDTA,
c) Aminoverbindungen der Formel

   R¹R²N - R - X

   wobei X = SO₃H, CH(NH₂)-CO₂H, CO₂H, H, NH₂ oder OH ist, R = C₁-C₉-Alkylen, C₃-C₆-Cycloalkylen oder Benzyliden ist und R¹ und R² voneinander unabhängig H oder C₁-C₃-Alkyl sind,
d) Guanidinobuttersäure,
e) Dimethylbiguanid,
f) Arginin,
g) Glucosamin, Fructose,
h) Pyrimidinnukleosiden und Pyrimidinnukleotiden,
i) mit einer oder mehreren Hydroxy-, Keto- oder/und weiteren Carboxygruppen substituierte Carbonsäuren
bestehenden Gruppe enthält.

Unter K1K2P pro gemäß vorliegender Erfindung versteht man ein nicht glykosyliertes t-PA-Derivat, das mit den Aminosäuren 85-92 beginnt und bei 527 (Pro) endet. K1K2P pro kann zusätzlich noch ganz oder teilweise die Aminosäuren -3(Gly) bis +5(Ile) enthalten. Dabei ist ein Protein bevorzugt, das bei Aminosäure 87(Asp) beginnt und ggf. aus dem Bereich -3 bis +5 noch die Aminosäuren Ser, Tyr, Gln, Val und Ile vorgeschaltet enthält (Nomenklatur nach Harris, Protein Engineering, Band 1 (1987) 449-458). Ein t-PA-Derivat K1K2P pro ist nach dem in der DE-39 233 391 beschriebenen Verfahren erhältlich.

Für die Solubilisierung von K1K2P pro ist ein Citrat-Puffer besonders geeignet. Die Konzentration der Citrationen soll mindestens 5 mmol/l, vorzugsweise von 5 bis 100 mmol/l betragen, besonders bevorzugt ist eine Konzentration der Citrationen von 50 mmol/l. Der pH-Wert wird je nach Basizität der zugesetzten Verbindung vorzugsweise mit HCl oder einer Base wie z.B. NaOH oder KOH eingestellt.

Überraschenderweise wurde festgestellt, daß die Löslichkeit von nicht glykosyliertem K1K2P pro in anderen Puffersystemen, z.B. Phosphatpuffer, bei gleicher Ionenstärke und gleichem pH-Wert wesentlich geringer ist. Es hat sich als geeignet erwiesen, den pH-Wert von alkalischen Citratlösungen mit HCl einzustellen, d.h. daß die Zusammensetzung zusätzlich noch Chloridionen enthält. In Gegenwart von Chloridionen sind nämlich überraschenderweise hochkonzentrierte Lösungen von K1K2P pro wesentlich stabiler als z.B. in Gegenwart von Phosphationen. Saure Citratlösungen werden üblicherweise mit NaOH auf den jeweiligen pH-Wert eingestellt.

Geeignet für eine erfindungsgemäße Zusammensetzung ist ein pH-Wert zwischen 4,5 und 6,5, bevorzugt ist ein pH-Wert von 6.

Vorzugsweise werden für eine erfindungsgemäße Zusammensetzung als Aminoverbindungen Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Aminobutan, 1,3-Aminopropan, Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure verwendet. Die bevorzugte Konzentration für Taurin und analoge Verbindungen beträgt 0,1 bis 0,5 mol/l, besonders bevorzugt 0,1 bis 0,3 mol/l. 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan werden vorzugsweise mit 10 bis 100 mmol/l eingesetzt. Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure werden vorzugsweise mit 0,5 bis 20 mmol/l, besonders bevorzugt mit 1 bis 10 mmol/l verwendet.

Als Carbonsäure, die mit einer oder mehreren Hydroxy-, Keto- oder/und weiteren Carboxygruppen substituiert ist, verwendet man z.B. Apfelsäure, Milchsäure, Fumarsäure oder Oxoglutarsäure. Diese Substanzen werden vorzugsweise mit 1 mmol/l bis 1000 mmol/l, besonders bevorzugt mit 10 bis 500 mmol/l verwendet.

Guanidinobuttersäure oder Arginin werden vorzugsweise mit 10 bis 200 mmol/l, besonders bevorzugt mit 50 bis 100 mmol/l verwendet. Für Dimethylbiguanid beträgt die Konzentration 50 bis 400 mmol/l, vorzugsweise 100 bis 300 mmol/l.

EDTA wird vorzugsweise mit 1 bis 200 mmol/l, besonders bevorzugt mit 10 bis 100 mmol/l verwendet. Ascorbinsäure wird vorzugsweise mit 0,1 bis 1 mol/l, besonders bevorzugt mit 0,2 bis 0,3 mol/l eingesetzt.

Glucosamin und Fructose werden bevorzugt in Konzentrationen von 1 bis 500 mmol/l, besonders bevorzugt mit 10 bis 300 mmol/l eingesetzt.

Als Pyrimidinnukleoside und Pyrimidinnukleotide sind z.B. Thymidin, Cytosin und Uridin bzw. die entsprechenden Nukleotide geeignet. Sie werden vorzugsweise in Konzentrationen von 1 bis 300 mmol/l, besonders bevorzugt 10 bis 300 mmol/l eingesetzt.

Weiterhin ein Gegenstand der Erfindung ist eine erfindungsgemäße Zusammensetzung, die zusätzlich eine oder mehrere Aminosäuren, insbesondere Histidin enthält.

Im folgenden ist eine Reihe besonders bevorzugter Präparate gemäß vorliegender Erfindung aufgeführt.

Eine Formulierung enthält 50 mmol/l Na-Citrat, pH 6 und 0,1 bis 0,3 mol/l Taurin. Bevorzugt ist auch eine Formulierung mit 50 mmol/l Na-Citrat, pH 6 und 0,2 bis 0,3 mol/l Ascorbinsäure.

Weiterhin bevorzugt ist eine Formulierung mit 50 mmol/l Na-Citrat/HCl, pH 6 und 1 mmol/l bis 10 mmol/l 7-Aminoheptansäure, 8-Aminooctansäure, p-Aminomethylbenzoesäure, ε-Aminocapronsäure, δ-Aminovaleriansäure, γ-Aminobuttersäure, Tranexamsäure, Lysin oder Ornithin.

Weiterhin besonders bevorzugt sind auch Formulierungen, die 50 mmol/l Na-Citrat/HCl, pH 6,0 und 50 bis 100 mmol/l Guanidinobuttersäure oder Arginin enthalten.

Bevorzugt ist auch eine Formulierung, die 50 mmol/l Na-Citrat, pH 6 und 10 bis 100 mmol/l EDTA enthält. Wiederum eine weitere Formulierung enthält 50 mmol/l NaCitrat/HCl, pH 6 und 100 bis 300 mmol/l Dimethylbiguanid.

Eine weitere Formulierung enthält 50 mmol/l NaCitrat/HCl, pH 6 und 10 bis 300 mmol/l Thymidin, Cytosin oder Uridin. Wiederum eine weitere Formulierung enthält 50 mmol/l NaCitrat/HCl, pH 6 und 10 bis 100 mmol/l 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan. Eine weitere Formulierung enthält 50 mmol/l NaCitrat/HCl, pH 6 und 10 bis 300 mmol/l Fructose oder Glucosamin.

Schließlich enthält eine weitere Formulierung 50 mmol/l NaCitrat, pH 6 und 10 bis 500 mmol/l Apfelsäure, Milchsäure, Fumarsäure oder 2-Oxoglutarsäure.

Auch Kombinationen aus mehreren der obengenannten Verbindungen mit Citrat bewirken eine sehr gute Löslichkeit von Proteinen mit t-PA-Aktivität.

Ein Gegenstand der Erfindung ist schließlich auch ein Arzneimittel auf Basis eines nicht-glykosylierten t-PA-Derivats K1K2P pro als Wirkstoff in Lösung oder als Lyophilisat mit den angegebenen Wirkstoffen und gegebenenfalls noch weiteren pharmazeutisch verträglichen Zusatz-, Hilfs-, Träger- und Füllstoffen.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Kronzentration, enthält. Derartige Zusätze sind beispielsweise Mannit, Tartrat- oder Citrat-Puffer, Ethanol, Komplexbildner wie z.B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die Erfindung die Verwendung von K1K2P pro zur Herstellung erfindungsgemäßer pharmazeutischer Präparate.

Die folgenden Beispiele sollen die konkrete Ausführung der Erfindung weiter erläutern.

### Beispiel 1

### Löslichkeit von K1K2P pro

Gereinigtes K1K2P pro (gelöst in 0.5 mol/l Arginin/H₃PO₄, pH 7,2) wird durch Ultrafiltration über eine YM 10-Membran (Amicon) konzentriert. Jeweils 0.5 ml des Konzentrats (Aktivität: 3.5 MU/ml) wird gegen die in Tabelle 1 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42 (1987), 478 - 486). Die Einheit U ist eine Einheit der Aktivität nach Definition der WHO, National Institute for Biological Standards and Control.

**Tabelle 1**

| Puffer | Aktivität | |
|---|---|---|
| | MU/ml | MU |
| 50 mmol/l NaCitrat/HCl, pH 6 | 0,08 | 0,05 |
| 50 mmol/l NaCitrat/HCl, pH 6 | 1,20 | 0,84 |
| 10 mmol/l ε-Aminocapronsäure | | |
| 50 mmol/l NaCitrat/NaOH, pH 6 | 1,56 | 0,94 |
| 0,3 mol/l Ascorbinsäure | | |
| 50 mmol/l NaCitrat/HCl, pH 6 | 0,81 | 0,49 |
| 10 mmol/l Lysin | | |
| 50 mmol/l NaCitrat/HCl, pH 6 | 0,71 | 0,46 |
| 50 mmol/l Arginin | | |
| 50 mmol/l NaCitrat/HCl, pH 6 | 1,10 | 0,77 |
| 50 mmol/l Guanidinobuttersäure | | |
| 50 mmol/l NaCitrat/NaOH, pH 6 | 0,80 | 0,56 |
| 100 mmol/l EDTA | | |
| 50 mmol/l Nacitrat/NaOH, pH 6 | 0,45 | 0,22 |
| 0,3 mol/l 2-Oxoglutarsäure | | |
| 50 mmol/l NaCitrat/HCl, pH 6 | 0,49 | 0,37 |
| 0,3 mol/l Glucosamin | | |
| 50 mmol/l NaCitrat/NaOH, pH 6 | 0,56 | 0,36 |
| 0,3 mol/l Fumarsäure | | |
| 50 mmol/l NaCitrat/HCl, pH 6 | 1,20 | 0,60 |
| 0,3 mol/l Uridin | | |
| 50 mmol/l NaCitrat/NaOH, pH 6 | 1,68 | 1,34 |
| 0,3 mol/l Taurin | | |
| 50 mmol/l NaCitrat/HCl, pH 6 | 0,42 | 0,27 |
| 0,1 mol/l Thymidin | | |

## Patentansprüche (Patentansprüche für folgende(n) Vertragsstaat(en): AT, BE, CH, LI, DE, DK, FR, GB, IT, LU, NL, SE)

1. Pharmazeutisches Präparat eines nicht glykosylierten t-PA-Derivats K1K2P pro mit einer enzymatischen Aktivität von mindestens 0,4 MU/ml und einem pH-Wert von 4,5 bis 6,5,
**dadurch gekennzeichnet,**
daß es Citrat und mindestens eine Verbindung aus der aus
a) Ascorbinsäure,
b) EDTA,
c) Aminoverbindungen der Formel
R¹R²N - R - X
wobei X = SO₃H, CH(NH₂)-CO₂H, CO₂H, H, NH₂ oder OH ist; R = C₁-C₉-Alkylen, C₃-C₆-Cycloalkylen oder Benzyliden ist; und R¹ und R² voneinander unabhängig H oder C₁-C₃-Alkyl sind,
d) Guanidinobuttersäure,
e) Dimethylbiguanid,
f) Arginin,
g) Glucosamin, Fructose,
h) Pyrimidinnukleosiden und Pyrimidinnukleotiden,
i) mit einer oder mehreren Hydroxy-, Keto- oder/und weiteren Carboxygruppen substituierte Carbonsäuren
bestehenden Gruppe enthält.

2. Pharmazeutisches Präparat nach Anspruch 1,
**dadurch gekennzeichnet,**
daß die Aminoverbindung Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminoheyan, 1,5-Diaminopentan, 1,4-Diaminobutan, 1,3-Diaminopropan, Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure ist.

3. Pharmazeutisches Präparat nach Anspruch 1,
**dadurch gekennzeichnet,**
daß die mit einer oder mehreren Hydroxy-, Keto- oder/und weiteren Carboxygruppen substituierte Carbonsäure Apfelsäure, Milchsäure, Fumarsäure oder 2-Oxoglutarsäure ist.

4. Pharmazeutisches Präparat nach Anspruch 1,
**dadurch gekennzeichnet,**
daß es zusätzlich eine oder mehrere Aminosäuren enthält.

5. Pharmazeutisches Präparat nach einem der Ansprüche 1 bis 4, **dadurch gekennzeichnet,** daß die Citrat-Konzentration 5 bis 100 mmol/l, vorzugsweise 50 mmol/l beträgt.

6. Pharmazeutisches Präparat nach einem der Ansprüche 1 bis 5, **dadurch gekennzeichnet,** daß es zusätzlich Chlorid-Ionen enthält.

7. Pharmazeutisches Präparat nach Anspruch 1,
**dadurch gekennzeichnet,**
daß es 50 mmol/l Natriumcitrat, pH 6, und 0,1 bis 1 mol/l, vorzugsweise 0,2 bis 0,3 mol/l Ascorbinsäure enthält.

8. Pharmazeutisches Präparat nach Anspruch 1,
**dadurch gekennzeichnet,**
daß es 50 mmol/l Natriumcitrat, pH 6, und 1 bis 200 mmol/l, vorzugsweise 10 bis 100 mmol/l EDTA enthält.

9. Pharmazeutisches Präparat nach Anspruch 1,
**dadurch gekennzeichnet,**
daß es 50 mmol/l Natriumcitrat, pH6, und 0,1 bis 0,5 mol/l, vorzugsweise 0,1 bis 0,3 mol/l Taurin enthält.

10. Pharmazeutisches Präparat nach Anspruch 1,
**dadurch gekennzeichnet,**
daß es 50 mmol/] Natriumcitrat/HCl, pH 6 und 0,5 bis 20 mmol/l Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure enthält.

11. Pharmazeutisches Präparat nach Anspruch 1,
**dadurch gekennzeichnet,**
daß es 50 mmol/l Natriumcitrat/HCl, pH 6, und 10 bis 100 mmol/l 4-Aminobutanol-1, 5-Aminopentanol-1, 6-Aminohexanol-1, 1,3-Diaminopropan, 1,4-Diaminobutan, 1,5-Diaminopentan, 1,6-Diaminohexan, 1,7-Diaminoheptan, 1,8-Diaminooctan oder 1,9-Diaminononan enthält.

12. Pharmazeutisches Präparat nach Anspruch 1,
**dadurch gekennzeichnet,**
daß es 50 mmol/l Natriumcitrat/HCl, pH 6, und 10 bis 200 mmol/l, vorzugsweise 50 bis 100 mmol/l Guanidinobuttersäure oder Arginin enthält.

13. Pharmazeutisches Präparat nach Anspruch 1,
**dadurch gekennzeichnet,**
daß es 50 mmol/l Natriumcitrat/HCl, pH 6, und 50 bis 400 mmol/l, vorzugsweise 100 bis 300 mmol/l Dimethylbiguanid enthält.

14. Pharmazeutisches Präparat nach Anspruch 1,
**dadurch gekennzeichnet,**
daß es 50 mmol/l Natriumcitrat/HCl, pH6, und 1 bis 500 mmol/l, vorzugsweise 10 bis 300 mmol/l Glucosamin oder Fructose enthält.

15. Pharmazeutisches Präparat nach Anspruch 1,
**dadurch gekennzeichnet,**
daß es 50 mmol/l Natriumcitrat/HCl, pH 6, und 1 bis 300 mmol/l, vorzugsweise 10 bis 300 mmol/l Thymidin, Cytosin oder Uridin enthält.

16. Pharmazeutisches Präparat nach Anspruch 1,
**dadurch gekennzeichnet,**
daß es 50 mmol/l Natriumcitrat, pH 6, und 0,001 bis 1 mol/l, vorzugsweise 0,01 bis 0,5 mol/l Apfelsäure, Milchsäure, Fumarsäure oder 2-Oxoglutarsäure enthält.

17. Pharmazeutisches Präparat nach Anspruch 1,
**dadurch gekennzeichnet,**
daß es 50 mmol/l Natriumcitrat, pH 6, und eine Kombination von Substanzen aus der Gruppe a) bis i) nach Anspruch 1 enthält.

18. Arzneimittel auf Basis eines nicht-glykosylierten t-PA-Derivats K1K2P pro als Wirkstoff,
**gekennzeichnet dadurch**
die Zusammensetzung nach einem der vorhergehenden Ansprüche, gegebenenfalls zusammen mit üblichen pharmazeutischen Zusatz-, Hilfs- oder/und Trägerstoffen.

19. Verfahren zur Herstellung eines pharmazeutischen Präparats nach einem der Ansprüche 1 bis 15,
**dadurch gekennzeichnet,**
daß man das nicht glykosylierte t-PA-Derivat K1K2P pro zusammen mit Citrat und mindestens einer Substanz aus der Gruppe a) bis i) nach Anspruch 1 in eine geeignete pharmazeutische Darreichungsform überführt.

20. Verfahren nach Anspruch 19, **dadurch gekennzeichnet,** daß die pharmazeutische Darreichungsform eine Injektionslösung oder ein Lyophilisat ist.

21. Verwendung des nicht glykosylierten t-PA-Derivats K1K2P pro zur Herstellung von pharmazeutischen Präparaten nach einem der Ansprüche 1 bis 18.

## Patentansprüche (Patentansprüche für folgende(n) Vertragsstaat(en): ES, GR)

1. Verfahren zur Herstellung eines pharmazeutischen Präparats eines nicht glykosilierten t-PA-Derivats K1K2P pro mit einer enzymatischen Aktivität von mindestens 0,4 MU/ml und einem pH-Wert von 4,5 bis 6,5,
**dadurch gekennzeichnet**,
daß man das t-PA-Derivat K1K2P pro zusammen mit Citrat und mindestens einer Verbindung aus der aus
a) Ascorbinsäure,
b) EDTA,
c) Aminoverbindungen der Formel
R¹R²N - R - X
wobei X = SO₃H, CH(NH₂)-CO₂H, CO₂H, H, NH₂ oder OH ist; R = C₁-C₉-Alkylen, C₃-C₆-Cycloalkylen oder Benzyliden ist; und R¹ und R² voneinander unabhängig H oder C₁-C₃-Alkyl sind,
d) Guanidinobuttersäure,
e) Dimethylbiguanid,
f) Arginin,
g) Glucosamin, Fructose,
h) Pyrimidinnukleosiden und Pyrimidinnukleotiden,
i) mit einer oder mehreren Hydroxy-, Keto- oder/und weiteren Carboxygruppen substituierte Carbonsäuren
bestehenden Gruppe in eine geeignete pharmazeutische Darreichungsform überführt.

2. Verfahren nach Anspruch 1,
**dadurch gekennzeichnet**,
daß die Aminoverbindung Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan, 1,3-Diaminopropan, Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure ist.

3. Verfahren nach Anspruch 1,
**dadurch gekennzeichnet**,
daß die mit einer oder mehreren Hydroxy-, Keto- oder/und weiteren Carboxygruppen substituierte Carbonsäure Apfelsäure, Milchsäure, Fumarsäure oder 2-Oxoglutarsäure ist.

4. Verfahren nach Anspruch 1,
**dadurch gekennzeichnet**,
daß man zusätzlich eine oder mehrere Aminosäuren hinzufügt.

5. Verfahren nach einem der Ansprüche 1 bis 4,
**dadurch gekennzeichnet**,
daß die Citrat-Konzentration 5 bis 100 mmol/l, vorzugsweise 50 mmol/l beträgt.

6. Verfahren nach einem der Ansprüche 1 bis 5,
**dadurch gekennzeichnet**,
daß man zusätzlich Chlorid-Ionen hinzufügt.

7. Verfahren nach Anspruch 1,
**dadurch gekennzeichnet**,
daß man 50 mmol/l Natriumcitrat, pH 6, und 0,1 bis 1 mol/l, vorzugsweise 0,2 bis 0,3 mol/l Ascorbinsäure verwendet.

8. Verfahren nach Anspruch 1,
**dadurch gekennzeichnet**,
daß man 50 mmol/l Natriumcitrat, pH 6, und 1 bis 200 mmol/l, vorzugsweise 10 bis 100 mmol/l EDTA verwendet.

9. Verfahren nach Anspruch 1,
**dadurch gekennzeichnet**,
daß man 50 mmol/l Natriumcitrat, pH 6, und 0,1 bis 0,5 mol/l, vorzugsweise 0,1 bis 0,3 mol/l Taurin verwendet.

10. Verfahren nach Anspruch 1,
**dadurch gekennzeichnet**,
daß man 50 mmol/l Natriumcitrat/HCl, pH 6, und 0,5 bis 20 mmol/l Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure verwendet.

11. Verfahren nach Anspruch 1,
**dadurch gekennzeichnet**,
daß man 50 mmol/l Natriumcitrat/HCl, pH 6, und 10 bis 100 mmol/l 4-Aminobutanol-1, 5-Aminopentanol-1, 6-Aminohexanol-1, 1,3-Diaminopropan, 1,4-Diaminobutan, 1,5-Diaminopentan, 1,6-Diaminohexan, 1,7-Diaminoheptan, 1,8-Diaminooctan oder 1,9-Diaminononan verwendet.

12. Verfahren nach Anspruch 1,
**dadurch gekennzeichnet**,
daß man 50 mmol/l Natriumcitrat/HCl, pH 6, und 10 bis 200 mmol/l, vorzugsweise 50 bis 100 mmol/l Guanidinobuttersäure oder Arginin verwendet.

13. Verfahren nach Anspruch 1,
**dadurch gekennzeichnet**,
daß man 50 mmol/l Natriumcitrat/HCl, pH 6, und 50 bis 400 mmol/l, vorzugsweise 100 bis 300 mmol/l Dimethylbiguanid verwendet.

14. Verfahren nach Anspruch 1,
**dadurch gekennzeichnet**,
daß man 50 mmol/l Natriumcitrat/HCl, pH 6, und 1 bis 500 mmol/l, vorzugsweise 10 bis 300 mmol/l Glucosamin oder Fructose verwendet.

15. Verfahren nach Anspruch 1,
**dadurch gekennzeichnet**,
daß man 50 mmol/l Natriumcitrat/HCl, pH 6, und 1 bis 300 mmol/l, vorzugsweise 10 bis 300 mmol/l Thymidin, Cytosin oder Uridin verwendet.

16. Verfahren nach Anspruch 1,
**dadurch gekennzeichnet**,
daß man 50 mmol/l Natriumcitrat, pH 6, und 0,001 bis 1 mol/l, vorzugsweise 0,01 bis 0,5 mol/l Apfelsäure, Milchsäure, Fumarsäure oder 2-Oxoglutarsäure verwendet.

17. Verfahren nach Anspruch 1,
**dadurch gekennzeichnet**,
daß man 50 mmol/l Natriumcitrat, pH 6, und eine Kombination von Substanzen aus der Grupe a) bis i) nach Anspruch 1 verwendet.

18. Verfahren nach einem der vorhergehenden Asprüche,
**dadurch gekennzeichnet**,
daß man außerdem übliche pharmazeutische Zusatz-, Hilfs- oder/und Trägerstoffe zusetzt.

19. Verfahren nach Anspruch 18,
**dadurch gekennzeichnet**,
daß die pharmazeutische Darreichungsform eine Injektionslösung oder ein Lyophilisat ist.

20. Verwendung des nicht glykosilierten t-PA-Derivats K1K2P pro zur Herstellung von pharmazeutischen Präparaten in einem Verfahren nach einem der Ansprüche 1 bis 18.

## Claims (Claims for the following Contracting State(s): AT, BE, CH, LI, DE, DK, FR, GB, IT, LU, NL, SE)

1. Pharmaceutical preparation of a non-glycosylated t-PA derivative K1K2P pro with an enzymatic activity of at least 0.4 MU/ml and a pH value of 4.5 to 6, characterised in that it contains citrate and at least one compound of the group consisting of
a) ascorbic acid,
b) EDTA,
c) amino compounds of the formula
R¹R²N - R - X
whereby X = SO₃H, CH(NH₂)-CO₂H, CO₂H, H, NH₂ or OH; R = C₁-C₉-alkylene, C₃-C₆-cycloalkylene or benzylidene; and R¹ and R², independently of one another, are H or C₁-C₃-alkyl,
d) guanidinobutyric acid,
e) dimethylbiguanide,
f) arginine,
g) glucosamine, fructose,
h) pyridimine nucleosides and pyrimidinenucleotides,
i) carboxylic acids substituted with one or more hydroxyl, keto and/or further carboxyl groups.

2. Pharmaceutical preparation according to claim 1, characterised in that the amino compound is taurine, 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane, 1,3-diaminopropane, lysine, ornithine, 8-aminooctanoic acid, 7-aminoheptanoic acid, ε-aminocaproic acid, δ-aminovaleric acid, γ-aminobutyric acid, tranexamic acid or p-aminomethylbenzoic acid.

3. Pharmaceutical preparation according to claim 1, characterised in that the carboxylic acid substituted with one or more hydroxyl, keto and/or further carboxyl groups is malic acid, lactic acid, fumaric acid or 2-oxoglutaric acid.

4. Pharmaceutical preparation according to claim 1, characterised in that it additionally contains one or more amino acids.

5. Pharmaceutical preparation according to one of claims 1 to 4, characterised in that the citrate concentration amounts to 5 to 100 mmol/l, preferably 50 mmol/l.

6. Pharmaceutical preparation according to one of claims 1 to 5, characterised in that it additionally contains chloride ions.

7. Pharmaceutical preparation according to claim 1, characterised in that it contains 50 mmol/l Na citrate, pH 6 and 0.1 to 1 mol/l, preferably 0.2 to 0.3 mol/l ascorbic acid.

8. Pharmaceutical preparation according to claim 1, characterised in that it contains 50 mmol/l Na citrate, pH 6, and 1 to 200 mmol/l, preferably 10 to 100 mmol/l EDTA.

9. Pharmaceutical preparation according to claim 1, characterised in that it contains 50 mmol/l Na citrate, pH 6, and 0.1 to 0.5 mol/l, preferably 0.1 to 0.3 mol/l taurine.

10. Pharmaceutical preparation according to claim 1, characterised in that it contains 50 mmol/l Na citrate/HCl, pH 6 and 0.5 to 20 mmol/l lysine, ornithine, 8-aminooctanoic acid, 7-aminoheptanoic acid, ε-aminocaproic acid, δ-aminovaleric acid, γ-aminobutyric acid, tranexamic acid or p-aminomethylbenzoic acid.

11. Pharmaceutical preparation according to claim 1, characterised in that it contains 50 mmol/l Na citrate/HCl, pH 6, and 10 to 100 mmol/l 4-aminobutanol-1, 5-aminopentanol-1, 6-aminohexanol-1, 1,3-diaminopropane, 1,4-diaminobutane, 1,5-diaminopentane, 1,6-diaminohexane, 1,7-diaminoheptane, 1,8-diaminooctane or 1,9-diaminononane.

12. Pharmaceutical preparation according to claim 1, characterised in that it contains 50 mmol/l Na citrate/HCl, pH 6, and 10 to 200 mmol/l, preferably 50 to 100 mmol/l guanidinobutyric acid or arginine.

13. Pharmaceutical preparation according to claim 1, characterised in that it contains 50 mmol/l Na citrate/HCl, pH 6, and 50 to 400 mmol/l, preferably 100 to 300 mmol/l dimethylbiguanide.

14. Pharmaceutical preparation according to claim 1, characterised in that it contains 50 mmol/l Na citrate/HCl, pH 6, and 1 to 500 mmol/l, preferably 10 to 300 mmol/l glucosamine or fructose.

15. Pharmaceutical preparation according to claim 1, characterised in that it contains 50 mmol/l Na citrate/HCl, pH 6, and 1 to 300 mmol/l, preferably 10 to 300 mmol/l thymidine, cytosine or uridine.

16. Pharmaceutical preparation according to claim 1, characterised in that it contains 50 mmol/l Na citrate, pH 6, and 0.001 to 1 mol/l, preferably 0.01 to 0.5 mol/l malic acid, lactic acid, fumaric acid or 2-oxoglutaric acid.

17. Pharmaceutical preparation according to claim 1, characterised in that it contains 50 mmol/l Na citrate, pH 6, and a combination of substances from the group a) to i) according to claim 1.

18. Medicament based on a non-glycosylated t-PA derivative K1K2P pro as active material, characterised by the composition according to one of the preceding claims, possibly together with usual pharmaceutical additive, adjuvant and/or carrier materials.

19. Process for the production of a pharmaceutical preparation according to one of claims 1 to 15, characterised in that one converts the non-glycosylated t-PA derivative K1K2P pro, together with citrate and at least one substance from the group a) to i) according to claim 1, into a suitable pharmaceutical form of administration.

20. Process according to claim 19, characterised in that the pharmaceutical form of administration is an injection solution or a lyophilisate.

21. Use of the non-glycosylated t-PA derivative K1K2P pro for the production of pharmaceutical preparations according to one of claims 1 to 18.

## Claims (Claims for the following Contracting State(s): ES, GR)

1. Process for the production of a pharmaceutical preparation of a non-glycosylated t-PA derivative K1K2P pro with an enzymatic activity of at least 0.4 MU/ml and a pH value of 4,5 to 6, characterised in that one converts the t-PA derivative K1K2P pro, together with citrate and at least one compound of the group consisting of
a) ascorbic acid,
b) EDTA
c) amino compounds of the formula
R¹R²N - R - X
whereby X = SO₃H, CH(NH₂)-CO₂H, CO₂H, H, NH₂ or OH; R = C₁-C₉-alkylene, C₃-C₆-cycloalkylene or benzylidene; and R¹ and R², independently of one another, are H or C₁-C₃-alkyl,
d) guanidinobutyric acid,
e) dimethylbiguanide,
f) arginine,
g) glucosamine, fructose,
h) pyrimidine nucleosides and pyrimidine nucleotides,
i) carboxylic acids substituted with one or more hydroxyl, keto and/or further carboxyl groups,
into a suitable pharmaceutical form of administration.

2. Process according to claim 1, characterised in that the amino compound is taurine, 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane, 1,3-diaminopropane, lysine, ornithine, 8-aminooctanoic acid, 7-aminoheptanoic acid, ε-aminocaproic acid, δ-aminovaleric acid, γ-aminobutyric acid, tranexamic acid or p-aminomethylbenzoic acid.

3. Process according to claim 1, characterised in that the carboxylic acid substituted with one or more hydroxyl, keto and/or further carboxyl groups is malic acid, lactic acid, fumaric acid or 2-oxoglutaric acid.

4. Process according to claim 1, characterised in that one additionally adds thereto one or more amino acids,

5. Process according to one of claims 1 to 4, characterised in that the citrate concentration amounts to 5 to 100 mmol/l, preferably 50 mmol/l.

6. Process according to one of claims 1 to 5, characterised in that one additionally adds thereto chloride ions.

7. Process according to claim 1, characterised in that one uses 50 mmol/l Na citrate, pH 6, and 0.1 to 1 mol/l, preferably 0.2 to 0.3 mol/l ascorbic acid,

8. Process according to claim 1, characterised in that one uses 50 mmol/l Na citrate, pH 6, and 1 to 200 mmol/l, preferably 10 to 100 mmol/l EDTA.

9. Process according to claim 1, characterised in that one uses 50 mmol/l Na citrate, pH 6, and 0.1 to 0.5 mol/l, preferably 0.1 to 0.3 mol/l taurine,

10. Process according to claim 1, characterised in that one uses 50 mmol/l Na citrate/HCl, pH 6, and 0.5 to 20 mmol/l lysine, ornithine, 8-aminooctanoic acid, 7-aminoheptanoic acid, ε-aminocaproic acid, δ-aminovaleric acid, γ-aminobutyric acid, tranexamic acid or p-aminomethylbenzoic acid,

11. Process according to claim 1, characterised in that one uses 50 mmol/l Na citrate/HCl, pH 6, and 10 to 100 mmol/l 4-aminobutanol-1, 5-aminopentanol-1, 6-aminohexanol-1, 1,3-diaminopropane, 1,4-diaminobutane, 1,5-diaminopentane, 1,6-diaminohexane, 1,7-diaminoheptane, 1,8-diaminooctane and 1,9-diaminononane.

12. Process according to claim 1, characterised in that one uses 50 mmol/l Na citrate/HCl, pH 6, and 10 to 200 mmol/l, preferably 50 to 100 mmol/l guanidinobutyric acid or arginine.

13. Process according to claim 1, characterised in that one uses 50 mmol/l Na citrate/HCl, pH 6, and 50 to 400 mmol/l, preferably 100 to 300 mmol/l dimethylbiguanide.

14. Process according to claim 1, characterised in that one uses 50 mmol/l Na citrate/HCl, pH 6, and 1 to 500 mmol/l, preferably 10 to 300 mmol/l glucosamine or fructose.

15. Process according to claim 1, characterised in that one uses 50 mmol/l Na citrate/HCl, pH 6, and 1 to 300 mmol/l, preferably 10 to 300 mmol/l thymidine, cytosine or uridine.

16. Process according to claim 1, characterised in that one uses 50 mmol/l Na citrate, pH 6, and 0.001 to 1 mol/l, preferably 0.01 to 0.5 mol/l malic acid, lactic acid, fumaric acid or 2-oxoglutaric acid.

17. Process according to claim 1, characterised in that one uses 50 mmol/l Na citrate, pH 6, and a combination of substances from the group a) to i) according to claim 1.

18. Process according to one of the preceding claims, characterised in that one additionally adds usual pharmaceutical additive, adjuvant and/or carrier materials.

19. Process according to claim 18, characterised in that the pharmaceutical form of administration is an injection solution or a lyophilisate.

20. Use of the non-glycosylated t-PA derivative K1K2P pro for the production of pharmaceutical preparations in a process according to one of claims 1 to 18.

## Revendications (Revendications pour l'(les) Etat(s) contractant(s) suivant(s): AT, BE, CH, LI, DE, DK, FR, GB, IT, LU, NL, SE)

1. Préparation pharmaceutique d'un dérivé K1K2P pro non glycosylé du t-PA ayant une activité enzymatique d'au moins 0,4 MU/ml et un pH de 4,5 à 6,5, caractérisée en ce qu'elle contient du citrate et au moins un-composé du groupe consistant en
a) l'acide ascorbique,
b) l'EDTA
c) des composés aminés de formule
R¹R²N - R - X
dans laquelle X représente SO₃H, CH(NH₂)-CO₂H, CO₂H, H, NH₂ ou OH; R représente un groupe alkylène en C₁-C₉, cycloalkylène en C₃-C₆ ou benzylidène, et R¹ et R² représentent indépendamment l'un de l'autre H ou un groupe alkyle en C₁-C₃,
d) l'acide guanidinobutyrique,
e) le diméthylbiguanide,
f) l'arginine,
g) la glucosamine, le fructose,
h) des nucléosides pyrimidiques et des nucléotides pyrimidiques,
i) des acides carboxyliques substitués avec un ou plusieurs groupes hydroxyle, cétoniques et/ou autres groupes carboxyle.

2. Préparation pharmaceutique selon la revendication 1, caractérisée en ce que le composé aminé est la taurine, le 4-aminobutan-1-ol, le 5-aminopentan-1-ol, le 6-aminohexan-1-ol, le 1,9-diaminononane, le 1,8-diaminooctane, le 1,7-diaminoheptane, le 1,6-diaminohexane, le 1,5-diaminopentane, le 1,4-diaminobutane, le 1,3-diaminopropane, la lysine, l'ornithine, l'acide 8-aminooctanoïque, l'acide 7-aminoheptanoïque, l'acide ε-aminocaproïque, l'acide δ-aminovalérique, l'acide γ-aminobutyrique, l'acide tranexamique ou l'acide p-aminométhylbenzoïque.

3. Préparation pharmaceutique selon la revendication 1, caractérisée en ce que l'acide carboxylique substitué avec un ou plusieurs groupes hydroxyle, cétoniques et/ou autres groupes carboxyle est l'acide malique, l'acide lactique, l'acide fumarique ou l'acide 2-oxoglutarique.

4. Préparation pharmaceutique selon la revendication 1, caractérisée en ce qu'elle contient en outre un ou plusieurs acides aminés.

5. Préparation pharmaceutique selon l'une des revendications 1 à 4, caractérisée en ce que la concentration en citrate est de 5 à 100 mmol/l, de préférence de 50 mmol/l.

6. Préparation pharmaceutique selon l'une des revendications 1 à 5, caractérisée en ce qu'elle contient en outre des ions chlorure.

7. Préparation pharmaceutique selon la revendication 1, caractérisée en ce qu'elle contient 50 mmol/l de citrate de sodium, pH 6, et 0,1 à 1 mol/l, de préférence 0,2 à 0,3 mol/l, d'acide ascorbique.

8. Préparation pharmaceutique selon la revendication 1, caractérisée en ce qu'elle contient 50 mmol/l de citrate de sodium, pH 6, et 1 à 200 mmol/l, de préférence 10 à 100 mmol/l, d'EDTA.

9. Préparation pharmaceutique selon la revendication 1, caractérisée en ce qu'elle contient 50 mmol/l de citrate de sodium, pH 6, et 0,1 à 0,5 mol/l, de préférence 0,1 à 0,3 mol/l, de taurine.

10. Préparation pharmaceutique selon la revendication 1, caractérisée en ce qu'elle contient 50 mmol/l de citrate de sodium/HCl, pH 6, et 0,5 à 20 mmol/l de lysine, d'ornithine, d'acide 8-aminooctanoïque, d'acide 7-aminoheptanoïque, d'acide ε-aminocaproïque, d'acide δ-aminovalérique, d'acide γ-aminobutyrique, d'acide tranexamique ou d'acide p-aminométhylbenzoïque.

11. Préparation pharmaceutique selon la revendication 1, caractérisée en ce qu'elle contient 50 mmol/l de citrate de sodium/HCl, pH 6, et 10 à 100 mmol/l de 4-aminobutan-1-ol, de 5-aminopentan-1-ol, de 6-aminohexan-1-ol, de 1,3-diaminopropane, de 1,4-diaminobutane, de 1,5-diaminopentane, de 1,6-diaminohexane, de 1,7-diaminoheptane, de 1,8-diaminooctane ou de 1,9-diaminononane.

12. Préparation pharmaceutique selon la revendication 1, caractérisée en ce qu'elle contient 50 mmol/l de citrate de sodium/HCl, pH 6, et 10 à 200 mmol/l, de préférence 50 à 100 mmol/l, d'acide guanidinobutyrique ou d'arginine.

13. Préparation pharmaceutique selon la revendication 1, caractérisée en ce qu'elle contient 50 mmol/l de citrate de sodium/HCl, pH 6, et 50 à 400 mmol/l, de préférence 100 à 300 mmol/l, de diméthylbiguanide.

14. Préparation pharmaceutique selon la revendication 1, caractérisée en ce qu'elle contient 50 mmol/l de citrate de sodium/HCl, pH 6, et 1 à 500 mmol/l, de préférence 10 à 300 mmol/l, de glucosamine ou de fructose.

15. Préparation pharmaceutique selon la revendication 1, caractérisée en ce qu'elle contient 50 mmol/l de citrate de sodium/HCl, pH 6, et 1 à 300 mmol/l, de préférence 10 à 300 mmol/l, de thymidine, de cytosine ou d'uridine.

16. Préparation pharmaceutique selon la revendication 1, caractérisée en ce qu'elle contient 50 mmol/l de citrate de sodium, pH 6, et 0,001 à 1 mol/l, de préférence 0,01 à 0,5 mol/l, d'acide malique, d'acide lactique, d'acide fumarique ou d'acide 2-oxoglutarique.

17. Préparation pharmaceutique selon la revendication 1, caractérisée en ce qu'elle contient 50 mmol/l de citrate de sodium, pH 6, et une combinaison de substances du groupe a) à i) selon la revendication 1.

18. Médicament à base d'un dérivé K1K2P pro non glycosylé du t-PA en tant que principe actif, caractérisé par la composition selon l'une des revendications précédentes, éventuellement en combinaison avec des additifs, adjuvants et/ou véhicules pharmaceutiques habituels.

19. Procédé d'obtention d'une préparation pharmaceutique selon l'une des revendications 1 à 15, caractérisé en ce que l'on convertit le dérivé K1K2P pro non glycosylé du t-PA en combinaison avec du citrate et au moins une substance du groupe a) à i) selon la revendication 1 en une forme d'administration pharmaceutique appropriée.

20. Procédé selon la revendication 19, caractérisé en ce que la forme d'administration pharmaceutique est une solution pour injection ou un lyophilisat.

21. Utilisation du dérivé K1K2P pro non glycosylé du t-PA pour l'obtention de préparations pharmaceutiques selon l'une des revendications 1 à 18.

## Revendications (Revendications pour l'(les) Etat(s) contractant(s) suivant(s): ES, GR)

1. Procédé d'obtention d'une préparation pharmaceutique d'un dérivé K1K2P pro non glycosylé du t-PA ayant une activité enzymatique d'au moins 0,4 MU/ml et un pH de 4,5 à 6,5, caractérisé en ce que l'on convertit le dérivé K1K2P pro non glycosylé du t-PA en combinaison avec du citrate et au moins un composé du groupe consistant en
a) l'acide ascorbique,
b) l'EDTA
c) des composés aminés de formule
R¹R²N - R - X
dans laquelle X représente SO₃H, CH(NH₂)-CO₂H, CO₂H, H, NH₂ ou OH; R représente un groupe alkylène en C₁-C₉, cycloalkylène en C₃-C₆ ou benzylidène, et R¹ et R² représentent indépendamment l'un de l'autre H ou un groupe alkyle en C₁-C₃,
d) l'acide guanidinobutyrique,
e) le diméthylbiguanide,
f) l'arginine,
g) la glucosamine, le fructose,
h) des nucléosides pyrimidiques et des nucléotides pyrimidiques,
i) des acides carboxyliques substitués avec un ou plusieurs groupes hydroxyle, cétoniques et/ou autres groupes carboxyle en une forme d'administration pharmaceutique appropriée.

2. Procédé selon la revendication 1, caractérisé en ce que le composé aminé est la taurine, le 4-aminobutan-1-ol, le 5-aminopentan-1-ol, le 6-amino-hexan-1-ol, le 1,9-diaminononane, le 1,8-diaminooctane, le 1,7-diaminoheptane, le 1,6-diaminohexane, le 1,5-diamino-pentane, le 1,4-diaminobutane, le 1,3-diaminopropane, la lysine, l'ornithine, l'acide 8-aminooctanoïque, l'acide 7-aminoheptanoïque, l'acide ε-aminocaproïque, l'acide δ-aminovalérique, l'acide γ-aminobutyrique, l'acide tranexamique ou l'acide p-aminométhylbenzoïque.

3. Procédé selon la revendication 1, caractérisé en ce que l'acide carboxylique substitué avec un ou plusieurs groupes hydroxyle, cétoniques et/ou autres groupes carboxyle est l'acide malique, l'acide lactique, l'acide fumarique ou l'acide 2-oxoglutarique.

4. Procédé selon la revendication 1, caractérisé en ce que l'on ajoute en outre un ou plusieurs acides aminés.

5. Procédé selon l'une des revendications 1 à 4, caractérisé en ce que la concentration en citrate est de 5 à 100 mmol/l, de préférence de 50 mmol/l.

6. Procédé selon l'une des revendications 1 à 5, caractérisé en ce que l'on ajoute en outre des ions chlorure.

7. Procédé selon la revendication 1, caractérisé en ce que l'on utilise 50 mmol/l de citrate de sodium, pH 6, et 0,1 à 1 mol/l, de préférence 0,2 à 0,3 mol/l, d'acide ascorbique.

8. Procédé selon la revendication 1, caractérisé en ce que l'on utilise 50 mmol/l de citrate de sodium, pH 6, et 1 à 200 mmol/l, de préférence 10 à 100 mmol/l, d'EDTA.

9. Procédé selon la revendication 1, caractérisé en ce que l'on utilise 50 mmol/l de citrate de sodium, pH 6, et 0,1 a 0,5 mol/l, de préférence 0,1 à 0,3 mol/l, de taurine.

10. Procédé selon la revendication 1, caractérisé en ce que l'on utilise 50 mmol/l de citrate de sodium/HCl, pH 6, et 0,5 à 20 mmol/l de lysine, d'ornithine, d'acide 8-aminooctanoïque, d'acide 7-amino-heptanoïque, d'acide ε-aminocaproïque, d'acide δ-amino-valérique, d'acide γ-aminobutyrique, d'acide tranexamique ou d'acide p-aminométhylbenzoïque.

11. Procédé selon la revendication 1, caractérisé en ce que l'on utilise 50 mmol/l de citrate de sodium/HCl, pH 6, et 10 à 100 mmol/l de 4-aminobutan-1-ol, de 5-aminopentan-1-ol, de 6-aminohexan-1-ol, de 1,3-diaminopropane, de 1,4-diaminobutane, de 1,5-diaminopentane, de 1,6-diaminohexane, de 1,7-diaminoheptane, de 1,8-diamino-octane ou de 1,9-diaminononane.

12. Procédé selon la revendication 1, caractérisé en ce que l'on utilise 50 mmol/l de citrate de sodium/HCl, pH 6, et 10 à 200 mmol/l, de préférence 50 à 100 mmol/l, d'acide guanidinobutyrique ou d'arginine.

13. Procédé selon la revendication 1, caractérisé en ce que l'on utilise 50 mmol/l de citrate de sodium/HCl, pH 6, et 50 à 400 mmol/l, de préférence 100 à 300 mmol/l, de diméthylbiguanide.

14. Procédé selon la revendication 1, caractérisé en ce que l'on utilise 50 mmol/l de citrate de sodium/HCl, pH 6, et 1 à 500 mmol/l, de préférence 10 à 300 mmol/l, de glucosamine ou de fructose.

15. Procédé selon la revendication 1, caractérisé en ce que l'on utilise 50 mmol/l de citrate de sodium/HCl, pH 6, et 1 à 300 mmol/l, de préférence 10 à 300 mmol/l, de thymidine, de cytosine ou d'uridine.

16. Procédé selon la revendication 1, caractérisé en ce que l'on utilise 50 mmol/l de citrate de sodium, pH 6, et 0,001 à 1 mol/l, de préférence 0,01 à 0,5 mol/l, d'acide malique, d'acide lactique, d'acide fumarique ou d'acide 2-oxoglutarique.

17. Procédé selon la revendication 1, caractérisé en ce que l'on utilise 50 mmol/l de citrate de sodium, pH 6, et une combinaison de substances du groupe a) à i) selon la revendication 1.

18. Procédé selon l'une des revendications précédentes, caractérisé en ce que l'on ajoute en outre des additifs, adjuvants et/ou véhicules pharmaceutiques habituels.

19. Procédé selon la revendication 18, caractérisé en ce que la forme d'administration pharmaceutique est une solution pour injection ou un lyophilisat.

20. Utilisation du dérivé K1K2P pro non glycosylé du t-PA pour l'obtention de préparations pharmaceutiques dans un procédé selon l'une des revendications 1 à 18.
